Europäisches Patentamt

European Patent Office

Office européen des brevets

(19)

(11) EP 0 798 266 B2

(12) NEUE EUROPÄISCHE PATENTSCHRIFT

(45) Veröffentlichungstag und Bekanntmachung des Hinweises auf die Entscheidung über den Einspruch:
**02.01.2002 Patentblatt 2002/01**

(51) Int Cl.[7]: **C01B 33/193**

(45) Hinweis auf die Patenterteilung:
**22.07.1998 Patentblatt 1998/30**

(21) Anmeldenummer: **97104764.2**

(22) Anmeldetag: **20.03.1997**

(54) **Fällungskieselsäure**

Precipitated silica

Silice précipitée

(84) Benannte Vertragsstaaten:
**AT BE CH DE DK ES FI FR GB GR IE IT LI LU MC NL PT SE**

(30) Priorität: **27.03.1996 DE 19612118**
**15.10.1996 DE 19642448**

(43) Veröffentlichungstag der Anmeldung:
**01.10.1997 Patentblatt 1997/40**

(73) Patentinhaber: **Degussa AG**
**40474 Düsseldorf (DE)**

(72) Erfinder:
• **Siray, Mustafa, Dr.**
**53127 Bonn (DE)**
• **Kuhlmann, Robert**
**50374 Erftstadt (DE)**
• **Storeck, Arnold, Dr.**
**60322 Frankfurt (DE)**
• **Neumüller, Mathias**
**63594 Hasselroth (DE)**

(56) Entgegenhaltungen:
EP-A- 0 139 754  EP-A- 0 535 943
WO-A-92/02454  WO-A-94/10087
DE-A- 4 423 493  US-A- 5 279 815

• **Schriftenreihe Pigmente, Nr. 9, 3.Auflage, Juni 1988**

EP 0 798 266 B2

## Beschreibung

Diese Erfindung betrifft eine Fällungskieselsäure, ein Verfahren zur Herstellung einer Fällungskieselsäure sowie ihre Verwendung bei der Herstellung von Zahnpasten und/oder Zahnpflegemitteln.

Synthetisch hergestellte Kieselsäuren spielen seit vielen Jahren als Bestandteil von Zahnpflegemitteln eine große Rolle. Sie werden dabei entweder als Putzkörper und/oder Verdickungsmittel zur Einstellung der rheologischen Eigenschaften der Zahnpasten eingesetzt. Es sind auch bifunktionelle Kieselsäuren bekannt, die sowohl als Abrasiv- und auch als Verdickungsmittel Verwendung finden.

Aus dem Dokument US-A 5,225,177 (Huber) ist ein Verfahren zur Herstellung einer Kieselsäure mit einer Oberfläche von 50 - 250 $m^2$/g, Partikelgröße von 11 μm, Brechungsindex 1,45 und Öl-Absorption von <125 bekannt.

Aus dem Dokument EP 0 308 165 (Unilever) ist ein Verfahren zur Herstellung einer Kieselsäure mit einer Oberfläche von 420-550 $m^2$/g, Partikelgröße von 2 - 20 μm, Brechungsindex 1,444 - 1,460 und Öl-Absorption von 70 - 140 bekannt.

Aus dem Dokument WO 94/10087 (Crosfield) ist ein Verfahren zur Herstellung einer Kieselsäure mit einer Oberfläche von 10 - 90 $m^2$/g, Partikelgröße von 5 - 15 μm, Brechungsindex 1,43 - 1,443, Öl-Absorption von 70 - 150 und mittlerem Porendurchmesser von 2 - 8 nm bekannt.

Aus dem Dokument EP 0 535 943 (Crosfield) ist ein Verfahren zur Herstellung einer Kieselsäure mit einer Oberfläche von 100 - 450 $m^2$/g, Partikelgröße von 5 - 15 μm, Brechungsindex 1,43 - 1,443, Öl-Absorption von 70 - 130 und mittlerem Porendurchmesser von 2 - 12 nm bekannt.

Aus den Prospektdaten der Firma Rhone-Poulenc sind die Kieselsäuren Tixosil 53 bzw. Tixosil 73 bekannt, die bei einem Brechungsindex von 1,46 einen RDA-Wert von 55 % bzw. bei einer Brechungszahl von 1,44 einen RDA-Wert von 110 % aufweisen.

Diese Kieselsäuren haben für bestimmte Anwendungen den Nachteil, daß sie bei niedrigen Brechungszahlen hohe RDA-Werte aufweisen.

Aus der Patentschrift DE-A 44 23 493 ist ebenfalls ein Verfahren zur Herstellung einer Kieselsäure mit einer BET-Oberfläche von 10 - 130 $m^2$/g, CTAB-Oberfläche von 10 - 70 $m^2$/g, mittlerer Teilchendurchmesser von 5 - 20 μm, Cu-Abrieb von 5 - 20 mg und einem Verdickungsverhalten von 300 - 3500 mPa.s bekannt.

In der Schriftenreihe Pigmente der Firma Degussa, Nummer 9, Synthetische Kieselsäuren in Zahnpasten, 3. Auflage vom Juni 1988, Seite 10, Abbildung 4 ist eine vergleichende Prüfung der Abrasivität von Sidenten nach der Kupferabrieb- und RDA-Methode graphisch dargelegt. Eine aus der DE-A 44 23 493 nach Beispiel 1 hergestellte Kieselsäure weist einen Cu-Abrieb von 17 mg (entspricht einem RDA-Wert von ca. 90 %) bei einem Brechungsindex von 1,455 auf.

Auch diese Kieselsäure hat für bestimmte Anwendungen den Nachteil, daß sie bei niedriger Brechungszahl einen hohen Cu-Abrieb bzw. RDA-Wert aufweist.

Es bestand daher die Aufgabe, eine Kieselsäure herzustellen, die diese Nachteile nicht aufweist.

Gegenstand der Erfindung ist eine Fällungskieselsäure, die eine BET-Oberfläche von 25 - 350 $m^2$/g, eine CTAB-Oberfläche von 25 - 150 $m^2$/g, eine DBP-Aufnahme von 150 - 300 g/100 g, einen mittleren Teilchendurchmesser von 5 - 20 μm, einen Cu-Abrieb in 10 %iger Glyzerindispersion von 1 - 3,6 mg und einen Brechungsindex von 1,440 - 1,451 aufweist.

Ein weiterer Gegenstand der Erfindung ist ein Verfahren zur Herstellung der Fällungskieselsäure, die eine BET-Oberfläche von 25 - 350 $m^2$/g, eine CTAB-Oberfläche von 25 - 150 $m^2$/g, eine DBP-Aufnahme von 150 - 300 g/100 g, einen mittleren Teilchendurchmesser von 5 - 20 μm, einen Cu-Abrieb in 10 %iger Glyzerindisperion von 1 - 3,6 mg und einen Brechungsindex von 1,440 - 1,451 aufweist, welches dadurch gekennzeichnet ist, daß man zu einer Alkalisilikatlösung mit einer Konzentration von 1,5 bis 23 g/l $Na_2O$ (Alkalisilikat: Gewichtsverhältnis $SiO_2$/$Na_2O$ = 3,2 bis 3,5), die Säurelösung und Alkalisilikatlösung mit bestimmter Konzentration und bestimmter Zulaufge-schwindigkeit unter Aufrechterhaltung einer Fälltemperatur zwischen 60 und 90 °C derart zugibt, daß ein Viskositätsanstieg nach spätestens 20 % der Fälldauer eintritt, und die Zugabe der Reaktionspartner erst dann beendet, wenn ein Kieselsäuregehalt von größer als 130 g/l erreicht wird, im Anschluß die Suspension durch weitere Säurezugabe auf pH-Wert <6 einstellt, den Feststoff durch Filtration abtrennt, wäscht, trocknet und vermahlt.

Die Alkalisilikatlösung, die zugegeben wird kann eine Konzentration von 100 bis 120 g/l $Na_2O$ aufweisen. Der Gehalt an $SiO_2$ kann 350 bis 370 g/l betragen.

Die Konzentration der Schwefelsäurelösung kann 45 bis 55 Gew.-% oder 90 bis 97 Gew.-% betragen.

Während der Fällung kann die Temperatur der Reaktionsmischung konstant gehalten werden. Ebenso kann während der Fällung die Konzentration an $Na_2O$, die in der Vorlage eingestellt worden ist, konstant gehalten werden.

Die Zulaufgeschwindigkeit wird für die Alkalisilikatlösung und die Schwefelsäurelösung so gesteuert, daß die gewünschten konstanten Bedingungen eingehalten werden können.

Zur Filtration kann Kammer-, Membranfilterpresse, Bandfilter oder Drehfilter oder eine Kombination von zwei der zuvor genannten Filter eingesetzt werden.

Zur Trocknung kann ein Umlauftrockner, Etagentrockner, Flashtrockner, Spinflashtrockner, Stromtrockner oder ähnlicher Trockner zum Einsatz kommen.

Der Filterkuchen kann, nachdem seine Viskosität herabgesetzt wurde, in einem Sprühtrockner mit oder ohne integriertem Fließbett mittels Atomizer, Einstoffdüse oder Zweistoffdüse zerstäubt und getrocknet werden.

Der Filterkuchen kann ohne vorheriger Veränderung der Viskosität einer Mahltrocknung unterzogen werden.

Für die Vermahlung der getrockneten Kieselsäure können Mühlen vom Typ Querstrommühle, Luftstrahlmühle oder Pendelmühle mit oder ohne eingebautem Sichter eingesetzt werden.

Die erfindungsgemäße Fällungskieselsäure kann als Abrasiv- oder Verdickungskomponente in Zahnpastenformulierungen eingesetzt werden. Bevorzugt kann man die erfindungsgemäß hergestellte Fällungskieselsäure als Putzkörper bei der Herstellung von wasserhaltigen Zahnpastenformulierungen mit guter Transparenz einsetzen.

**Beispiele**

### Beispiel 1

In einem 30 l Behälter werden 0,37 l Natriumsilikatlösung (7,85 Gew.% $Na_2O$; 26,80 Gew.% $SiO_2$ mit Modul $SiO_2/Na_2O = 3,4$; Dichte 1,352 g/ml) und 18 l heißes Wasser auf 85°C eingestellt. Der $Na_2O$ Gehalt der Vorlagenlösung beträgt 2,1 g/l.

Während 157 Minuten werden gleichzeitig unter Konstanthalten der $Na_2O$-Konzentration von 2,1 g/l und der Temperatur von 85°C 14,7 l Natriumsilikatlösung und 3,28 l 50 %iger Schwefelsäure in die Vorlage unter intensivem Rühren zugegeben. Der Viskositätsanstieg erfolgt nach der 25. Minute.

Anschließend wird die Zufuhr an Natriumsilikat gestoppt und die Zufuhr der 50 %igen Schwefelsäure fortgesetzt bis ein pH-Wert in der Kieselsäuresuspension von 3,5 erreicht wird. Der Feststoffgehalt der Suspension liegt bei 156 g $SiO_2$/l.

Die Fällungskieselsäuresuspension wird anschließend filtriert und mit Wasser ausgewaschen.

Nach der Verflüssigung des Filterkuchens wird das Produkt in einem Sprühtrockner getrocknet und anschließend auf einer Labor-Stiftmühle vermahlen.

### Beispiel 2

In einem 30 l Behälter werden 1,13 l Natriumsilikatlösung (7,85 Gew.% $Na_2O$; 26,80 Gew.% $SiO_2$ mit Modul $SiO_2/Na_2O = 3,4$; Dichte 1,352 g/ml) und 15,6 l heißes Wasser auf 85°C eingestellt. Der $Na_2O$ Gehalt der Vorlagenlösung beträgt 7,15 g/l.

Während 98 Minuten werden gleichzeitig unter Konstanthalten der $Na_2O$-Konzentration von 7,15 g/l und der Temperatur von 85°C 8,97 l Natriumsilikatlösung und 1,915 l 50%iger Schwefelsäure in die Vorlage unter intensivem Rühren zugegeben. Der Viskositätsanstieg erfolgt nach der 23. Minute.

Anschließend wird die Zufuhr an Natriumsilikat gestoppt und die Zufuhr der 50 %igen Schwefelsäure fortgesetzt bis ein pH-Wert in der Kieselsäuresuspension von 3,5 erreicht wird. Der Feststoffgehalt der Suspension liegt bei 135 g $SiO_2$/l.

Die Fällungskieselsäuresuspension wird anschließend filtriert und mit Wasser ausgewaschen.

Nach der Verflüssigung des Filterkuchens wird das Produkt in einem Sprühtrockner getrocknet und anschließend auf einer Labor-Stiftmühle vermahlen.

### Beispiel 3

in einem 30 l Behälter werden 1,13 l Natriumsilikatlösung (7,85 Gew.% $Na_2O$; 26,80 Gew.% $SiO_2$ mit Modul $SiO_2/Na_2O = 3,4$ Dichte 1,352 g/ml) und 15,6 l heißes Wasser auf 85°C eingestellt. Der $Na_2O$ Gehalt der Vorlagenlösung beträgt 7,15 g/l.

Während 120 Minuten werden gleichzeitig unter Konstanthalten der $Na_2O$ -Konzentration von 7,15 g/l und der Temperatur von 85°C 11,17 l Natriumsilikatlösung und 2,375 l 50 %iger Schwefelsäure in die Vorlage unter intensivem Rühren zugegeben. Der Viskositätsanstieg erfolgt nach der 21. Minute.

Anschließend wird die Zufuhr an Natriumsilikat gestoppt und die Zufuhr der 50 %igen Schwefelsäure fortgesetzt bis ein pH-Wert in der Kieselsäuresuspension von 3,5 erreicht wird. Der Feststoffgehalt der Suspension liegt bei 150 g $SiO_2$/l.

Die Fällungskieselsäuresuspension wird anschließend filtriert und mit Wasser ausgewaschen.

Nach der Verflüssigung des Filterkuchens wird das Produkt in einem Sprühtrockner getrocknet und anschließend auf einer Labor-Stiftmühle vermahlen.

*Beispiel 4*

In einem 30 l Behälter werden 2,14 l Natriumsilikatlösung (7,85 Gew.% $Na_2O$; 26,80 Gew.% $SiO_2$ mit Modul $SiO_2/Na_2O = 3,4$; Dichte 1,352 g/ml) und 14,42 l heißes Wasser auf 85°C eingestellt. Der $Na_2O$ Gehalt der Vorlagenlösung beträgt 13,7 g/l.

Während 120 Minuten werden gleichzeitig unter Konstanthalten der $Na_2O$-Konzentration von 13,7 g/l und der Temperatur von 85°C 9,04 l Natriumsilikatlösung und 1,72 l 50 %iger Schwefelsäure in die Vorlage unter intensivem Rühren zugegeben. Der Viskositätsanstieg erfolgt nach der 25. Minute.

Anschließend wird die Zufuhr an Natriumsilikat gestoppt und die Zufuhr der 50 %igen Schwefelsäure fortgesetzt bis ein pH-Wert in der Kieselsäuresuspension von 3,5 erreicht wird. Der Feststoffgehalt der Suspension liegt bei 149 g $SiO_2$/l.

Die Fällungskieselsäuresuspension wird anschließend filtriert und mit Wasser ausgewaschen.

Nach der Verflüssigung des Filterkuchens wird das Produkt in einem Sprühtrockner getrocknet und anschließend auf einer Labor-Stiftmühle vermahlen.

*Beispiel 5*

In einem 1000 l Behälter werden 9,5 l Natriumsilikatlösung (7,85 Gew.% $Na_2O$; 26,80 Gew.% $SiO_2$ mit Modul $SiO_2/Na_2O = 3,4$; Dichte 1,352 g/ml) und 600 l heißes Wasser auf 65°C eingestellt. Der $Na_2O$ Gehalt der Vorlagenlösung beträgt 1,65 g/l.

Während 165 Minuten werden gleichzeitig unter Konstanthalten des sich bei der $Na_2O$-Konzentration von 1,65 g/l ergebenden pH-Wertes von 10,9 und Temperatur von 65°C 588,4 l Natriumsilikatlösung und 41,65 l 96%iger Schwefelsäure in die Vorlage unter intensivem Rühren zugegeben. Der Viskositätsanstieg erfolgt nach der 23. Minute.

Anschließend wird die Zufuhr an Natriumsilikat gestoppt und die Zufuhr der 96%igen Schwefelsäure fortgesetzt bis ein pH-Wert in der Kieselsäuresuspension von 3,5 erreicht wird. Der Feststoffgehalt der Suspension liegt bei 178 g $SiO_2$/l.

Die Fällungskieselsäuresuspension wird anschließend filtriert und mit Wasser ausgewaschen.

Nach der Verflüssigung des Filterkuchens wird das Produkt in einem Sprühtrockner getrocknet und anschließend auf einer Labor-Stiftmühle vermahlen.

Die physikalisch-chemischen und anwendungstechnischen Daten der erhaltenen Fällungskieselsäure sind in der Tabelle 1 aufgeführt.

Tabelle 1

| Beispiel | | 1 | 2 | 3 | 4 | 5 |
|---|---|---|---|---|---|---|
| | | | | | | |
| BET-Oberfläche | $m^2$/g | 113 | 221 | 209 | 200 | 215 |
| CTAB-Oberfläche | $m^2$/g | 77 | 92 | 89 | 60 | 87 |
| Mittlerer Teilchendurchmesser | µm | 10,2 | 10,5 | 10,5 | 11,8 | 13,0 |
| Cu-Abrieb | mg | 1 | 2,3 | 1,0 | 2,9 | 3,6 |
| Brechungsindex | | 1,45 | 1,448 | 1,45 | 1,449 | 1,448 |
| Feuchte | % | 3,8 | 5,6 | 4,8 | 4,9 | 5,5 |
| DBP-Aufnahme | g/100g | 189 | 223 | 203 | 206 | 194 |
| CMC-Verdickung | mPa s | 7200 | 29000 | 17000 | 7700 | 3100 |
| RDA | | | | | 12,6 | 19,8 |
| Gesamtfälldauer* | min | 178 | 129 | 151 | 192 | 227 |
| Feststoffgehalt Fällsuspension | g/l | 156 | 135 | 150 | 149 | 178 |
| Prozentualer Anteil Viskositätsanstieg an Gesamtfälldauer | % | 14 | 18 | 14 | 13 | 10 |

* Die Gesamtfälldauer setzt sich zusammen aus Fällzeit und Ansäuerungszeit bis zum End-pH-Wert.

Die Daten werden mittels der folgenden Methoden bestimmt:

- Die Bestimmung der spezifischen Stickstoff-Oberfläche (BET) erfolgt nach Brunauer-Emmet -Teller mit Hilfe der AREA-meter-Apparatur der Fa. Ströhlein. Die Bestimmung erfolgt gemäß DIN ISO 5794/1 Annex D. Die Originalmethode wurde erstmals in Journal of the American Chemical Society, 60 (1938) Seite 309 beschrieben. Die Ausheiztemperatur beträgt 160 °C für 1 Stunde.

- Die CTAB-Oberfläche wird durch Adsorption von Cetyltrimethylammoniumbromid bei pH 9 bestimmt (s. Jay, Janzen und Kraus in "Rubber Chemistry and Technology" 44 (1971) 1287).

- Die Teilchenverteilung wird mittels Coulter Counter, Modell TA II der Fa. Coulter Electronics, ermittelt. Zum Einsatz kommt die 100 μm-Kapillare.

- Die Bestimmung der Abrasivität erfolgt nach der Cu-Abriebsmethode in 10 %iger Glyzerindispersion (153 g Glyzerin wasserfrei, in dem 17 g Kieselsäure 12 min bei 1 500 UpM mit dem Flügelrührer dispergiert werden). Die Abriebmessung erfolgt durch 50 000 Doppelhübe mittels Nylonbürsten an Cu-Blechen (Elektrolyt-Kupfer) in obiger Dispersion. Aus der Differenzwägung erhält man die mg Cu-Abrieb.
Literatur: Pfrengle, Fette, Seifen, Anstrichmittel 63 (1961) 445-451 und Reng, Dany, Parfümerie und Kosmetik 59 (1978) 37-45.

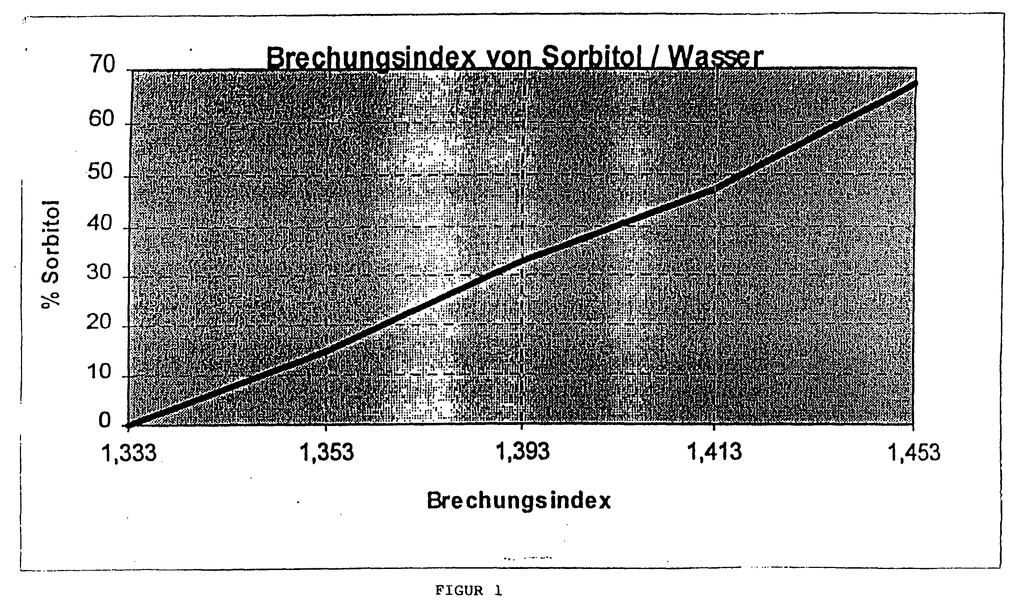

- Die Bestimmung des Brechungsindexes wird wie folgt durchgeführt:
147,0 g 70 %iges Sorbitol und 3 g Kieselsäure oder Silikat werden in ein 150 ml Titriergefäß aus Braunglas eingewogen. Das Titriergefäß wird mit einem Deckel verschlossen und 3 Tage stehen gelassen, um eine völlige Benetzung zu garantieren. Die Titration erfolgt mit entionisiertem Wasser und als Sensor dient eine Phototrode. Diese wird zunächst mit entionisiertem Wasser auf 1000 mV = 100 % Transparenz eingestellt. Die Bürette wird gespült und die Methoden-Nummer eingegeben. Dann wird das Titriergefäß an den Titrierkopf des Titrators geschraubt. Vor der eigentlichen Titration wird 3 Minuten gerührt, um die Probe gut zu dispergieren. Dann wird entionisiertes Wasser in 0,5 ml Schritten zugegeben und zwischen jeder Zugabe 9 Sekunden gerührt. Das Maximalvolumen ist auf 40 ml eingestellt. Nach dieser Zugabe wird die Titrierkurve und die Meßwerttabelle ausgedruckt. Der ml-Verbrauch wird aus der Titrierkurve im Wendepunkt (Maximum) oder alternativ aus der Meßwerttabelle (Figur 1) abgelesen. Hieraus errechnet sich die Sorbitolkonzentration.
Berechnung:

$$\% \text{ Sorbitol} = \frac{E \cdot K \cdot 100}{(E + V) \cdot 100}$$

E = Einwaage Sorbitol in g
K = Sorbitol-Konzentration in % (z.B. 70)
V = ml Wasser (d = 1)

- Die Bestimmung des Verdickungsverhaltens erfolgt 20 %ig in einer Carboxymethylcellulose-Lösung (50 g PEG 400, 1 kg 87 %iges Glyzerin, 25 g AKU CMC LZ 855, 925 g Wasser). Die mindestens 1 Tag aber höchstens 2 Wochen alte Testlösung wird mit Kieselsäure versetzt, dispergiert und die Viskosität bestimmt (Brookfield RVT, Spindel 5, 10 UpM, Wert nach 1 min). Die Messung der bei 25 °C thermostatisierten Mischung erfolgt sofort, nach 0,5 h und nach 24 h. Letzterer ist der eigentliche Meßwert.

- Daneben werden die Feuchte (2 h, 105 °C, DIN ISO 787 Teil 2), die Leitfähigkeit (4 %ig), der Glühverlust (2 h bei 1.000 °C, analog DIN 55 921) bestimmt.

**Patentansprüche**

1. Fällungskieselsäure, die eine BET-Oberfläche von 25 - 350 $m^2$/g, eine CTAB-Oberfläche von 25 - 150 $m^2$/g, eine DBP-Aufnahme von 150 - 300 g/100 g, einen mittleren Teilchendurchmesser von 5 - 20 μm, einen Cu-Abrieb in 10 %iger Glyzerindispersion von 1 - 3,6 mg und einen Brechungsindex von 1,440 - 1,451 aufweist.

2. Verfahren zur Herstellung einer Fällungskieselsäure gemäß Anspruch 1, die eine BET-Oberfläche von 25 - 350 $m^2$/g aufweist, eine CTAB-Oberfläche von 25-150 $m^2$/g, eine DBP-Aufnahme von 150 - 300 g/100 g, einen mittleren Teilchendurchmesser von 5 - 20 μm, einen Cu-Abrieb in 10 %iger Glyzerindispersion von 1 - 3,6 mg und einen Brechungsindex von 1,440 - 1,451 aufweist, **dadurch gekennzeichnet, daß** man zu einer Alkalisilikatlösung mit einer Konzentration von 1,5 bis 23 g/l $Na_2O$ (Alkalisilikat: Gewichtsverhältnis $SiO_2/Na_2O$ = 3,2 - 3,5) Säurelösung und Alkalisilikatlösung mit bestimmter Konzentration und bestimmter Zulaufgeschwindigkeit unter Aufrechterhaltung einer Fälltemperatur zwischen 60 und 90 °C derartig zugibt, daß ein Viskositätsanstieg nach spätestens 20 % der Gesamtfälldauer eintritt, und die Zugabe der Reaktionspartner erst dann beendet, wenn ein Kieselsäuregehalt von größer als 130 g/l erreicht ist, im Anschluß die Suspension durch weitere Säurezugabe auf pH-Wert <6 einstellt, den Feststoff durch Filtration abtrennt, wäscht, trocknet und vermahlt.

3. Verfahren gemäß Anspruch 2, **dadurch gekennzeichnet, daß** man zur Filtration Kammerfilterpessen oder Membranfilterpressen, oder Bandfilter, oder Drehfilter, oder zwei der genannten Filter in Kombination einsetzt.

4. Verfahren gemäß Anspruch 2, **dadurch gekennzeichnet, daß** man zur Trocknung einen Umlauftrockner, Etagentrockner, Stromtrockner, Flash-Trockner, Spinflash-Trockner oder ähnliche Einrichtungen einsetzt.

5. Verfahren gemäß Anspruch 2, **dadurch gekennzeichnet, daß** man verflüssigten Filterkuchen in einem Sprühtrockner mit Atomizer oder Zweistoffdüse oder Einstoffdüse und/oder integriertem Fließbett trocknet.

6. Verfahren gemäß Anspruch 2, **dadurch gekennzeichnet, daß** man den Filterkuchen einer Mahltrocknung unterzieht.

7. Verfahren gemäß Anspruch 2, **dadurch gekennzeichnet, daß** man das getrocknete Pulver mittels Querstrommühle, Pendelmühle, Luftstrahlmühle mit oder ohne eingebautem Sichter vermahlt.

8. Verwendung der Fällungskieselsäure nach Anspruch 1 zur Herstellung von Zahnpasten und/oder Zahnpflegemitteln.

## Claims

1. Precipitated silica which has a BET surface area of 25 - 350 $m^2/g$, a CTAB surface area of 25 - 150 $m^2/g$, a DBP absorption of 150 - 300 g/100 g, an average particle diameter of 5 - 20 um, a Cu abrasion in 10 % strength glycerine dispersion of 1 - 3,6 mg and a refractive index of 1.440 - 1.451.

2. Process for preparing a precipitated silica in accordance with Claim 1, which has a BET surface area of 25 - 350 $m^2/g$, a CTAB surface area of 25 - 150 $m^2/g$, a DBP absorption of 150 - 300 g/100 g, an average particle diameter of 5 - 20 um, a Cu abrasion in 10 % strength glycerine dispersion of 1 - 3,6 mg and a refractive index of 1.440 - 1.451, **characterised in that** an acid solution and an alkali metal silicate solution, with a specific concentration and a specific rate of flow, is added to an alkali silicate solution with a concentration of 1.5 to 23 g/l of $Na_2O$ (alkali metal silicate: ratio by wt. $SiO_2/Na_2O$ = 3.2 - 3.5), while maintaining a precipitation temperature between 60 and 90°C, in such a way that an increase in viscosity takes place at the latest after 20 % of the entire precipitation time, and addition of the reaction partners is ended when a silica content of greater than 130 g/l has been reached, then the pH of the suspension is adjusted to <6 by adding further acid and the solid is isolated by filtration, washed, dried and milled.

3. Process according to Claim 2, **characterised in that** chamber filter presses or membrane filter presses, or a belt filter or a rotary filter, or a combination of two of the filters mentioned are used for the filtration procedure.

4. Process according to Claim 2, **characterised in that** a circulation dryer, multiplate dryer, air-lift dryer, flash dryer, spin-flash dryer or a similar device is used for the drying procedure.

5. Process according to Claim 2, **characterised in that** the liquefied filter cake is dried in a spray drier with an atomiser or two-fluid nozzle or single-fluid nozzle and/or an integrated fluidised bed.

6. Process according to Claim 2, **characterised in that** the filter cake is subjected to mill drying.

7. Process according to Claim 2, **characterised in that** the dried powder is milled by means of a cross-flow mill, pendulum roller mill, compressed air mill with or without a built-in air separator.

8. Use of precipitated silica according to Claim 1 to prepare toothpaste and/or dental care products.

**Revendications**

1. Acide silicique de précipitation qui possède :

   - une surface BET de 25 à 350 m$^2$/g
   - une surface CTAB de 25 à 150 m$^2$/g
   - une adsorption DBP de 150 à 300 g/100 g
   - un diamètre moyen de particules de 5 à 20 μm
   - une perte à la friction du cuivre dans une dispersion de glycérol à 10 % de 1 à 3,6 mg et,
   - un indice de réfraction de 1,440 à 1,451.

2. Procédé de production d'un acide silicique de précipitation conformément à la revendication 1 qui possède une surface BET de 25 à 350 m$^2$/g qui possède une surface CTAB de 25 à 150 m$^2$/g, une adsorption DBP de 150-300 g/100g, un diamètre moyen de particules de 5 à 20 μm, une perte à la friction du cuivre en dispersion à 10 % de glycérol de 1 à 3,6 mg et un indice de réfraction de 1,440 à 1,451,
   **caractérisé en ce qu'**
   on ajoute à une solution de silicate de métal alcalin ayant une concentration de 1,5 à 23 g/l de Na$_2$O (silicate alcalin : rapport pondérai SiO$_2$/Na$_2$O = 3,2-3,5) une solution d'acide et une solution de silicate de métal alcalin ayant une concentration déterminée et avec une vitesse d'admission déterminée tout en maintenant une température de précipitation entre 60 et 90°C, de telle façon qu'une montée de la viscosité survienne après au plus tard 20 % de la durée totale de précipitation et que l'addition des partenaires de réaction soit achevée dans ce cas seulement lorsqu'une teneur supérieure à 130 g/l est obtenue, comme suite la suspension est ajustée à une valeur de pH < 6 par addition supplémentaire d'acide, on sépare la matière solide par filtration, on lave, on sèche et on broie.

3. Procédé selon la revendication 2,
   **caractérisé en ce qu'**
   on met en oeuvre pour la filtration des presses à filtre à plateaux ou des presses à filtre à membrane, ou des filtres à bandes, ou des filtres rotatifs ou bien deux des filtres cités, en combinaison.

4. Procédé selon la revendication 2,
   **caractérisé en ce qu'**
   on met en oeuvre pour le séchage, un séchoir à circulation, un séchoir à étages, un séchoir à courant, un séchoir flash, un séchoir tournant-flash ou des dispositifs similaires.

5. Procédé conformément à la revendication 2,
   **caractérisé en ce qu'**
   on sèche le gâteau de filtre fluidifié dans un séchoir par pulvérisation avec un atomiseur ou une buse pour deux produits ou une buse pour un seul produit et/ou avec un lit fluidifié intégré.

6. Procédé conformément à la revendication 2,
   **caractérisé en ce qu'**
   on soumet le gâteau de filtre à un séchage par broyage.

7. Procédé conformément à la revendication 2,
   **caractérisé en ce qu'**
   on broie la poudre séchée à l'aide d'un broyeur à courant transversal, un broyeur à cyllindres centrifugeuses, un broyeur à jet d'air avec ou sans dépoussiéreur incorpore.

8. Utilisation de l'acide silicique de précipitation selon la revendication 1 en vue de la production de pâtes dentifrices et/ou de produits d'hygiène dentaire.

## Brechungsindex von Sorbitol / Wasser

FIGUR 1

EP 0 798 266 B2